# EUROPEAN PATENT APPLICATION

(11) **EP 1 287 835 A1**
(43) Date of publication of application: **05.03.2003**
(21) Application number: 01120342.9
(22) Date of filing: 24.08.2001
(51) Int. Cl.: A61L 15/28

(54) **Chitosan-coated web and process for making the same**

(71) Applicant: THE PROCTER & GAMBLE COMPANY, Cincinnati, Ohio 45202 (US)
(72) Inventor: Tamburro, Maurizio, 65100 Pescara (IT); D'Alesio, Nicola, 66010 Canosa Sannita (Chieti) (IT); Pesce, Antonella, 65120 Pescara (IT); Di Cintio, Achille, 65126 Pescara (IT); Carlucci, Giovanni, 66100 Chieti (IT); Tordone, Adelia, 65125 Pescara (IT)
(74) Representative: Kremer, Véronique

(57) **Abstract**

The present invention relates to a particulate chitosan coated web for use in disposable absorbent articles and a process for making the same. The chitosan particles have a mean diameter of not more than 300 microns. The process involves the step applying onto the surface of a precursor web a solution or a dispersion comprising chitosan material in the form of a spray of droplets having a mean diameter of less than 1500 microns.

## Description

### FIELD OF THE INVENTION

The present invention relates to a chitosan-coated web for use in disposable absorbent articles and a process for making the same.

### BACKGROUND OF THE INVENTION

Webs, particularly fibrous structures for absorbing fluids, are manufactured for many uses. They are for example incorporated into absorbent articles such as disposable diapers, incontinent pads, sanitary napkins and panty liners as fluid absorption and/or fluid transmission and/or diffusion elements, especially as absorbent cores that are intended to absorb and retain body fluids.

The primary focus of absorbent articles is the ability of these articles to absorb and retain fluids. Besides this primary characteristic, other features like comfort are increasingly important for users of absorbent articles. To satisfy this need so-called breathable absorbent articles, offering gas and vapour exchange through the backsheet of the articles, have been developed and commercialised. However, breathable articles suffer from negatives like occurrence of undesired body fluid leakage both through the backsheet and along the peripheral article edges.

Attempts have been made to overcome this recurring problem, by for example increasing the amount of superabsorbent materials or using coagulants, see for example EP-A-906 074. However, these solutions are not completely overcoming this problem so that there still exists the need for alternative and improved solutions for the prevention of fluid leakage through the breathable backsheet of disposable absorbent articles in an easy and cost-effective manner.

It is therefore an object of the present invention to provide a web suitable for use in disposable absorbent articles offering effective fluid leakage prevention. More particularly it is an object of the present invention to provide a web to be used as absorbent core or so-called secondary backsheet in disposable absorbent articles, typically articles for feminine protection, which prevents body fluid leakage and wet-through the so-called primary breathable backsheet or outer backsheet of absorbent articles.

The above-mentioned objects have now surprisingly been met by providing a web comprising in at least one region particles of chitosan materials, having a particle size distribution with a mean diameter D(v,0.9) of not more than about 300 µm, preferably in a substantially homogeneous pattern as well as a process for making the chitosan-coated web.

Indeed, it has now been found that by selecting a particular active, namely chitosan material, with particular physical characteristics namely with a particle size distribution as indicated above, improved leakage prevention is obtained. This benefit is further improved by applying the particles in a particular manner onto/into a web, namely in a substantially homogeneous pattern.

Without wishing to be bound by any theory, it is believed that chitosan material retains electrolyte-containing fluids like body fluids by multiple mechanisms.

One mechanism is conventional absorption by incorporation of the water dipole molecules into the structure. As the quaternary ammonium groups, being positively charged, are distracting each other, molecular cavities exist, in which water molecules can penetrate. By the penetration of dipole molecules, like water, these cavities are widened by swelling and thereby generating even more space for further water molecules. This mechanism can be continued until the limits of molecular tension are reached.

The second mechanism of binding electrolyte-containing fluids, like body fluids, by chitosan material is gelification. Chitosan material acts electrostatically on nearby negatively charged molecules and thereby holds them in its circumference. The positively charged cationic groups (e.g., quaternary ammonium groups) of the chitosan material will interact with negatively charged anionic function-bearing molecules present in bodily fluids, like for example the carboxylic groups of proteins. This will result in the formation of a three-dimensional network between the chitosan material and such molecules with anionic groups (gelification of the bodily fluids). This gelification will further entrap other molecules present in body fluids (like lipids, acids).

Due to the gelification properties of the chitosan material with respect to electrolyte-containing fluids, a liquid barrier is generated when the chitosan material is wetted by such fluids.

Advantageously, the outstanding leakage prevention benefits associated to the present invention are further due to the selected particle size distribution of the particles of chitosan material. Indeed, without to be bound by theory, it is speculated that the higher active surface area of the small particles of chitosan materials according to the present invention, compared to bigger particles for a same total weight, contributes to improved activity of such materials. Advantageously, due to the high active surface area of the small chitosan material particles according to the present invention, the gelification and retention properties of chitosan material towards electrolyte-containing fluids are improved and hence the leakage prevention is significantly improved, this while using significantly less total amount of chitosan material.

In a preferred embodiment herein, further improved leakage benefit is provided to a web by applying the particles of chitosan materials in a substantially homogeneous pattern. Applying chitosan particles in an homogenous pattern more preferably in continuous pattern results in the formation of a gelifying film layer of chitosan material, which is substantially impervious to body fluids and hence prevents any occurrence of wetting through said web. This layer is not only able to absorb body fluids but also distribute said body fluids in the plane of the web, which is formed between the x- and y-axis of said web. Thus, the whole region covered by particles of chitosan material is utilized for body fluid gelification in a continuous manner. In other words, an exhaustive barrier for body fluids is formed.

In a particularly preferred embodiment, the web of the present invention is made of substantially hydrophilic material for optimum liquid absorption, liquid acquisition and/or liquid handling. This is particularly important when the web of the present invention is used as absorbent core or secondary backsheet in absorbent articles.

The present invention also encompasses a process of making an absorbent web, wherein a solution or dispersion of chitosan material is applied onto said web in the form of a spray of droplets, said droplets having a particle size distribution with a mean diameter D(v,0.9) of less than 1500 µm. An advantage of this process is that a homogeneous layer of the particles of chitosan materials is provided on the surface of the web, which translates in outstanding leakage prevention towards fluids while requiring a lower amount of actives. Indeed, by applying the solution or dispersion of chitosan materials onto the web in the form of a spray of small droplets as defined herein, a higher coverage can be achieved with the same amount of solution/dispersion as compared to applying the same solution/dispersion in the form of a spray of droplets with larger droplets. Furthermore, applying the solution/dispersion onto the web as mentioned herein further translates in limited wetting of the surface and thus in faster process ability of the web and subsequent disposable absorbent articles comprising the same.

### PRIOR ART BACKGROUND

The use of chitosan in absorbent articles has been discussed in several prior art documents. EP 627 225 discloses the preparation of chitosan compounds with improved absorption characteristics and suggests their usage in sanitary hygiene articles. DE 19,913,478 discloses a breast pad comprising chitosan for improved absorption of fat-containing liquids, such as milk. WO 99/61079 and WO 99/32697 disclose the use of chitosan coatings onto hydrophobic substrates for providing antimicrobial absorbent structures, e.g. nonwovens. EP 393 825 teaches the utilization of chitosan salts in absorbent products. A structure formed by a cellulose web containing chitosan for water absorbance and starch as the binder for the structure is disclosed.

None of the cited prior art discloses a web comprising at least one region comprising very small particles of chitosan material, preferably in a substantially homogeneous pattern, let alone the benefits associated thereby, namely leakage reduction or even prevention and improved body fluid handling and -absorption at reduced consumption of chitosan material.

### SUMMARY OF THE INVENTION

The present invention relates to a web suitable for use in disposable absorbent articles, said web having at least one region comprising particles of chitosan material with a particular particle size distribution, preferably in a substantially homogeneous pattern.

The present invention also encompasses a process for producing such a web, comprising the steps of forming the web and applying a solution or dispersion of chitosan material in the form of a spray of small droplets. A highly preferred embodiment requires the steps of applying latex binder onto the web before applying the solution or dispersion of chitosan material in the form of spray of small droplets.

### BRIEF DESCRIPTION OF THE DRAWINGS

Figure 1 illustrates one possible configuration of the process for making the web according to the present invention.
Figure 2 shows an exemplary ESEM (Environmental Scanning electronic microscope) picture (30x magnification) of a surface of a preferred fibrous airlaid web according to the present invention, having applied latex onto it and chitosan material on top of the latex. A substantially homogeneous pattern of chitosan material, forming a film-like layer, is clearly to be recognized.
Figure 3 shows an exemplary ESEM picture (20,000x magnification), of a preferred web according to the present invention wherein the particle size of the chitosan material applied to the web and the homogeneity of the applied pattern of chitosan particles is clearly to be recognized.

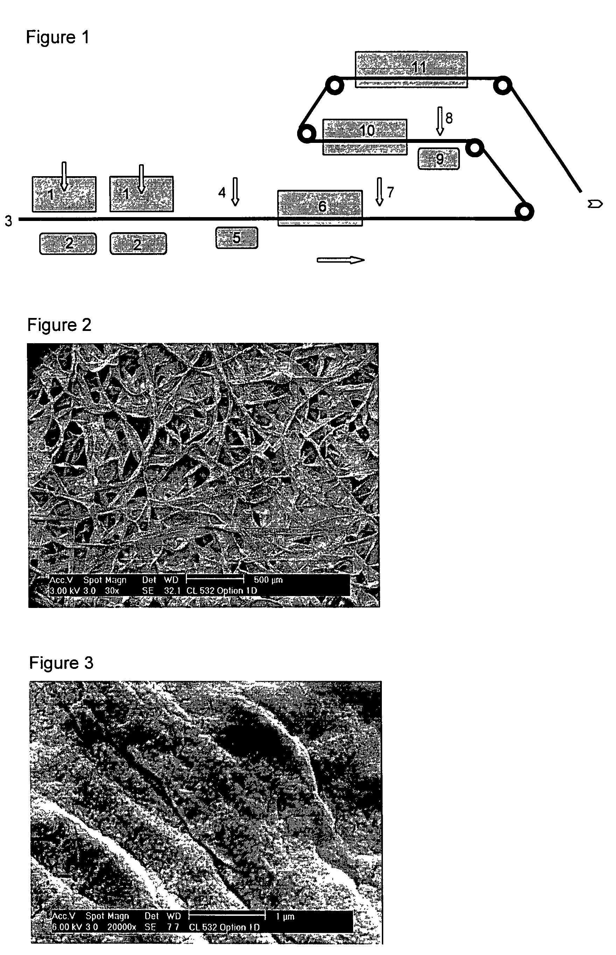

The Figures 2 and 3 were taken from a Philips XL30 ESEM FEG apparatus.

### DETAILED DESCRIPTION OF THE INVENTION

### Definitions

The term 'absorbent article' is used herein in a very broad sense including any article able to receive and/or absorb and/or contain and/or retain fluids and/or exudates, especially bodily fluids/bodily exudates. The absorbent article, which is referred to in the present invention typically comprises a structure having a fluid pervious topsheet, a fluid impervious backsheet that is preferably water vapour and/or gas pervious and an absorbent core element comprised there between. Particularly preferred absorbent articles in the context of the present invention are disposable absorbent articles. Typical disposable absorbent articles according to the present invention are diapers, incontinence pads, sanitary napkins, panty liners, tampons, interlabial devices, surgical and wound dressings and perspiration pads.

The term 'disposable' is used herein to describe articles, which are not intended to be laundered or otherwise restored or reused as an article (i.e. they are intended to be discarded after a single use and preferably to be recycled, composted or otherwise disposed of in an environmentally compatible manner).

The term 'use', as used herein, refers to the period of time that starts when the absorbent article is actually put in contact with the anatomy of the user.

### Web for use in absorbent articles

The present invention relates to a web, having two surfaces approximately aligned opposite to each other, for use in an absorbent article. In at least one region, said web comprises particles of chitosan material with a particular particle size distribution as described herein after.

In a preferred embodiment, the web of the present invention is used in breathable absorbent articles. In this embodiment it is preferred that the region of the web of the present invention, which comprises chitosan material, is directed away from the wearer's skin towards the garment of the wearer. In other words, the chitosan material-comprising region is directed towards the garment-facing surface of the breathable absorbent article (i.e., is at closer distance to the garment-facing surface of the breathable article than to the wearer-facing surface thereof). By this construction, a fluid barrier towards the backsheet of the absorbent article is established, which by gelification hinders or even prevents body fluids from approaching at the breathable backsheet. Another benefit of the web of the present invention is the prevention of leakage through the topsheet and along the peripheral edge of the article due to immobilization of body fluids by gelification.

The term 'web' is used herein to describe materials suitable for use in absorbent articles. The web of the present invention has a dimension along each of the x-, y-and z-axes of the orthogonal Cartesian system of coordinates. The web comprises two surfaces aligned substantially opposite to each other. The first surface extends in the plane formed between said x- and said y-axis of said web and the second surface is aligned approximately opposite to said first surface, the first and the second surface being spaced apart from each other by said z-dimension of the web. At least one region of said web comprises particles of chitosan material, preferably in a substantially homogeneous pattern. The web of the present invention can comprise a single layer or multiple layers.

Examples of webs for use in the present invention are fibrous structures, such as nonwovens or fabrics, comprising natural or synthetic fibres or mixtures thereof, or apertured polymeric films or foam materials. Indeed, the web of the present invention can be made of any of a variety of fibres, including a blend or admixture. The fibres may be cellulosic, modified cellulosic, or hydrophilic synthetic and include such fibres as wood pulp, rayon, cotton, cellulose acetate, polyester, nylon and the like. The web can be made according to any suitable method known for this purpose in the art. Fibrous webs according to the present invention can be made by appropriate processes such as dry laying and in particular air laying, melt blowing or spunbonding. Film-like or foam-like webs according to the present invention are made by processes suitable for such purposes.

Highly preferred webs for use herein are hydrophilic fibrous webs. As used herein, 'hydrophilic' refers to a material having a contact angle of water in air of less than 90 degrees, whereas the term 'hydrophobic' herein refers to a material having a contact angle of water in air of 90 degrees or greater. A web comprising hydrophilic fibres like for example cellulosic fibres such as wood pulp fibres is particularly useful as an absorbent structure in such products as sanitary napkins, disposable diapers or wipes because the hydrophilic fibres are liquid absorbent and therefore enhance the overall absorbency of the web. Preferably, webs for use herein can be made of a blend of cellulosic and hydrophilic synthetic fibres, typically comprising about 65% to 95% by weight of cellulosic fibres and more preferably up to about 20% by weight of the hydrophilic synthetic fibres. The hydrophilic synthetic fibres, which can be provided in any length including staple length, can improve the strength of the web. Hydrophobic fibres or films, such as fibres or films made of polyethylene or polypropylene, may also be used in the web herein provided they are treated by e.g. surfactants to make them hydrophilic, in order not to decrease the absorbent capacity of the preferred web, when incorporating them into said web.

In a preferred embodiment the web for use herein is a dry laid, preferably an air laid fibrous web. 'Dry laying' and more specifically, 'air laying' processes are widely used to produce webs from dry fibres, which can in turn be used e.g. as webs for absorbing fluids. Particularly, dry laying refers to e.g. carding or air laying. Carding refers to the formation of carded webs, i.e. webs in which the fibres are oriented (carded) in a given direction, whereas the air laying process refers to the formation of webs with a completely random fibre orientation; the properties of such air laid webs are therefore somewhat isotropic. The webs produced by dry laying processes are soft, flexible and porous and are particularly suitable for use as liquid absorbent structures in absorbent articles, such as disposable diapers, sanitary napkins, incontinent pads and wipes.

The dry laid manufacturing process generally comprises a web formation and layering step and a web bonding and stabilizing step; in dry laying processes in fact the fibres, that can be of any type, e.g. cellulosic, synthetic, or any combination thereof, are formed or condensed into a web, but such web lacks integrity and must therefore be stabilized. Different techniques for bonding and stabilizing a dry formed web are known in the art, i.e. mechanical, thermal and chemical bonding processes. Bonding a web structure by means of a chemical agent is one of the most common methods of bonding in the nonwoven industry and consists in the application of a chemical binder to the web and in the curing of the binder.

The web according to the present invention comprises as an essential component a chitosan material or a mixture thereof. By 'chitosan material', it is meant herein chitosans, modified chitosans, crosslinked chitosans or chitosan salts.

Chitosan is a partially or fully deacetylated form of chitin, a naturally occurring polysaccharide. Indeed, chitosan is an aminopolysaccharide usually prepared by deacetylation of chitin (poly-beta(1,4)-N-acetyl-D-glucosamine).

Chitosan is not a single, definite chemical entity but varies in composition depending on the conditions of manufacture. It may be equally defined as chitin sufficiently deacetylated to form soluble amine salts. Chitosan is the beta-(1,4)-polysaccharide of D-glucosamine and is structurally similar to cellulose, except that the C-2 hydroxyl group in cellulose is substituted with a primary amine group in chitosan. The large number of free amine groups makes chitosan a polymeric weak base. Solutions of chitosan are generally highly viscous, resembling those of natural gums.

The chitosan used herein is suitably in relatively pure form. Methods for the manufacture of pure chitosan are well known. Generally, chitin is milled into a powder and demineralised with an organic acid such as acetic acid. Proteins and lipids are then removed by treatment with a base, such as sodium hydroxide, followed by chitin deacetylation by treatment with concentrated base, such as 40 percent sodium hydroxide. The chitosan formed is washed with water until the desired pH is reached.

The properties of chitosan relate to its polyelectrolyte and polymeric carbohydrate character. Thus, it is generally insoluble in water, in alkaline solutions at pH levels above about 7, or in hydrophobic organic solvents. It generally dissolves readily in dilute aqueous solutions of organic acids such as formic, acetic, tartaric, glycolic, lactic and citric acids and also in dilute aqueous solutions of mineral acids, except, for example, sulphuric acid. In general, the amount of acid required to dissolve chitosan is approximately stoichiometric with the amino groups. Since the pKₐ for the amino groups present in chitosan is between 6.0 and 7.0, they can be protonated in very dilute acids or even close to neutral conditions, rendering a cationic nature to this biopolymer. This cationic nature is the basis of many of the benefits of chitosan. Indeed, chitosan material can be considered as a linear polyelectrolyte with a high charge density which can interact with negatively charged surfaces, like proteins (e.g. by interfering with the proteinic wall construction of microorganisms, thereby acting as an antimicrobial agent and/or by reacting with the proteins present in bodily fluid, like menses, thereby acting as a gelifying agent for such fluid).

Preferred chitosan materials for use herein have an average degree of deacetylation (D.A.) of more than 70%, preferably from 80% to about 100%. The degree of deacetylation refers to the percentage of the amine groups that are deacetylated. This characteristic is directly related to the hydrogen bonding existing in this biopolymer, affecting its structure, solubility and ultimately its reactivity. The degree of deacetylation can be determined by titration, dye adsorption, UV/vis, IR and NMR spectroscopy. The degree of deacetylation will influence the cationic properties of chitosan. By increasing the degree of deacetylation the cationic character of the chitosan material will increase and thus also its gelifying abilities.

Suitable chitosan materials to use herein include substantially water-soluble chitosan. As used herein, a material will be considered water-soluble when it substantially dissolves in excess water to form a clear and stable solution, thereby, losing its initially particulate form and becoming essentially molecularly dispersed throughout the water solution. Preferred chitosan materials for use herein are water soluble, i.e. at least 1 gram and preferably at least 3 gram of the chitosan materials are soluble in 100 grams of water at 25°C and one atmosphere. By 'solubility' of a given compound it is to be understood herein the amount of said compound solubilised in deionised water at 25°C and one atmosphere in absence of a precipitate. Generally, the water-soluble chitosan materials will be free from a higher degree of crosslinking, as crosslinking tends to render the chitosan materials water insoluble.

Chitosan materials (i.e. chitosan and chitosan salts, modified chitosans and cross-linked chitosans) may generally have a wide range of molecular weights. Chitosan materials with a wide range of molecular weights are suitable for use in the present invention. Typically, chitosan materials for use herein have a molecular weight ranging from 1,000 to 10,000,000 grams per gram moles and more preferably from 2,000 to 1,000,000. Molecular weight means average molecular weight. Methods for determining the average molecular weight of chitosan materials are known to those skilled in the art. Typical methods include for example light scattering, intrinsic viscosity and gel permeation chromatography. It is generally most convenient to express the molecular weight of a chitosan material in terms of its viscosity in a 1.0 weight percent aqueous solution at 25°C with a Brookfield viscometer. It is common to indirectly measure the viscosity of the chitosan material by measuring the viscosity of a corresponding chitosan salt, such as by using a 1.0 weight percent acetic acid aqueous solution. Chitosan materials suitable for use in the present invention will suitably have a viscosity in a 1.0 weight-% aqueous solution at 25°C of from about 10 mPa·s (10 centipoise) to about 100,000 mPa·s (100,000 centipoise), more suitably from about 30 mPa·s (30 centipoise) to about 10,000 mPa·s (10,000 centipoise), even more suitably 7000 mPa·s (7000 centipoise).

The pH of the chitosan materials depends on their preparation. Preferred chitosan materials for use herein have an acidic pH, typically in the range of 3 to 7, preferably about 5. By pH of the chitosan material, it is meant herein the pH of a 1% chitosan material solution (1 gram of chitosan material dissolved in 100 grams of distilled water) measured by a pH-meter. By using a more acidic pH, the cationic character of the chitosan materials will be increased and thus their gelifying abilities. However, too high acidity is detrimental to skin safety. Thus it is highly preferred herein to use chitosan materials with a pH of about 5, thereby delivering the best compromise between fluid handling properties on one side and skin compatibility on the other side.

Particularly suitable chitosan materials for use herein are chitosan salts, especially water-soluble chitosan salts. A variety of acids can be used for forming chitosan salts. Suitable acids for use are soluble in water or partially soluble in water, are sufficiently acidic to form the ammonium salt of chitosan and yet not sufficiently acidic to cause hydrolysis of chitosan and are present in amount sufficient to protonate the reactive sites of chitosan.

Preferred acids can be represented by the formula:

R-(COOH)ₙ

wherein n has a value of 1 to 3 and R represents a mono- or divalent organic radical composed of carbon, hydrogen and optionally at least one of oxygen, nitrogen and sulphur or simply R is an hydrogen atom. Preferred acids are the mono- and dicarboxylic acids composed of carbon, hydrogen, oxygen and nitrogen (also called hereinafter amino acids). Such acids are highly desired herein as they are biologically acceptable for use against or in proximity to the human body. Illustrative acids, in addition to those previously mentioned include, among others, are citric acid, formic acid, acetic acid, N-acetylglycine, acetylsalicylic acid, fumaric acid, glycolic acid, iminodiacetic acid, itaconic acid, lactic acid, maleic acid, malic acid, nicotinic acid, 2-pyrrolidone-5-carboylic acid, salycilic acid, succinamic acid, succinic acid, ascorbic acid, aspartic acid, glutamic acid, glutaric acid, malonic acid, pyruvic acid, sulfonyldiacetic acid, benzoic acid, epoxysuccinic acid, adipic acid, thiodiacetic acid and thioglycolic acid. Any chitosan salts formed from the reaction of chitosan with any of these acids are suitable for use herein.

Examples of chitosan salts formed with an inorganic acid include, but are not limited to, chitosan hydrochloride, chitosan hydrobromide, chitosan phosphate, chitosan sulphonate, chitosan chlorosulphonate, chitosan chloroacetate and mixtures thereof. Examples of chitosan salts formed with an organic acid include, but are not limited to, chitosan formate, chitosan acetate, chitosan lactate, chitosan glycolate, chitosan malonate, chitosan epoxysuccinate, chitosan benzoate, chitosan adipate, chitosan citrate, chitosan salicylate, chitosan propionate, chitosan nitrilotriacetate, chitosan itaconate, chitosan hydroxyacetate, chitosan butyrate, chitosan isobutyrate, chitosan acrylate and mixtures thereof. It is also suitable to form a chitosan salt using a mixture of acids including, for example, both inorganic and organic acids.

Highly preferred chitosan salts for use herein are those formed by the reaction of chitosan with an amino acid. Amino acids are molecules containing both an acidic and amino functional group. The use of amino acids is highly preferred as those chitosan amino salts have higher skin compatibility. Indeed most of the amino acids are naturally present on the skin. Chitosan salts of pyrrolidone carboxylic acid are effective moisturizing agents and are non-irritating to skin. Amino acids for use herein include both linear and/or cyclo amino acids. Examples of amino acids for use herein include, but are not limited to, alanine, valine, leucine, isoleucine, prolinephenylalanine, triptofane, metionine, glycine, serine, cysteine, tyrosine, asparagine, glutamine, aspartic acid, glutamic acid, lysine, arginine, istydine, hydroxyproline and the like. A particularly suitable example of a cyclic amino acid is pyrrolidone carboxylic acid, which is a carboxylic acid of pyrrolidin-2-one as per following formula:

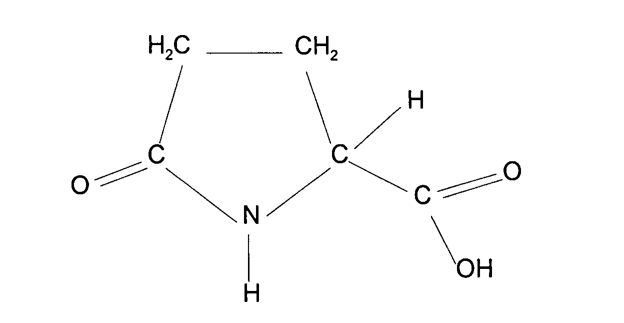

Other chitosan materials suitable for use herein include cross-linked chitosans with a low degree of cross-linkage and modified chitosans. Suitable crosslinking agents for use herein are organic compounds having at least two functional groups or functionalities capable of reacting with active groups located on the chitosan materials. Examples of such active groups include, but are not limited to, carboxylic acid (-COOH), amino (-NH₂), or hydroxyl (-OH) groups. Examples of such suitable crosslinking agents include, but are not limited to, diamines, polyamines, diols, polyols, dicarboxylic acids, polycarboxylic acids, aminocarboxylic acids, aminopolycarboxylic acids, polyoxides and the like. One way to introduce a crosslinking agent with the chitosan material solution is to mix the crosslinking agent with chitosan during preparation of the solution. Another suitable crosslinking agent comprises a metal ion with more than two positive charges, such as Ca²⁺, Al³⁺, Fe³⁺, Ce³⁺, Ce⁴⁺, Ti⁴⁺, Zr⁴⁺ and Cr³⁺. Since the cations on chitosan possess antimicrobial properties, it is preferred herein to not use a crosslinking agent reacting to the cations, unless no alternative crosslinking agent is available.

Modified chitosans for use herein are any chitosans where the glucan chains carry pendant groups. Examples of such modified chitosans include carboxymethyl chitosan, methyl pyrrolidinone chitosan, glycol chitosan and the like. Methyl pyrrolidone chitosan is for instance described in US 5,378,472. Water-soluble glycol chitosan and carboxymethyl chitosan are for instance described in WO 87/07618. Particularly suitable modified chitosans for use herein include water soluble covalently bonded chitosan derivatives or ionically bonded chitosan derivatives obtained by contacting salt of chitosan with electrophilic organic reagents. Such water-soluble chitosan derivatives are described in EP 737 692.

Examples of chitosan derivatives suitable for use herein are described in depth in EP 737 692.

Suitable chitosan material is commercially available from numerous vendors. Exemplary of a commercially available chitosan materials are those available from for example the Vanson Company. The preferred chitosan salt for use herein is chitosan pyrrolidone carboxylate (also called chitosonium pyrrolidone carboxylate), which has a degree of deacetylation of more than 85%, a water solubility of 1% (1 gram is soluble in 100 grams of distilled water at 25°C and one atmosphere) and a pH of about 5. Chitosonium pyrrolidone carboxylate is commercially available under the name Kytamer® PC from Amerchol Corporation.

Another preferred chitosan salt for use herein is chitosan lactate, the chitosan salt of lactic acid, which is commercially available from Vanson Company, Redmond, WA, USA.

As an essential feature, the chitosan material is present in at least one region of the web in the form of particles of chitosan material having a particular particle size distribution.

By 'region comprising particles of chitosan material', it is meant hereinafter any area located onto or within the web which comprises particles of a chitosan material. It is understood herein that such a region can comprise a fraction of the total web or might comprise the total web per se. For example in absorbent articles, especially those for feminine protection, like sanitary napkins or panty liners, such webs when used as absorbent cores and/or as secondary backsheet, comprise chitosan material in the so-called central region, i.e., a region where body fluids like menstruation is discharged in use, and/or in longitudinal and/or lateral zones, i.e. on the peripheral edges of the absorbent articles, where run-off leakage of liquid needs to be prevented. These regions can have any size or shape. Such a region can have the same extension in the plane formed between the x- and y-axis of the web as the web itself or only a fraction of this extension. Said regions can have an regular or irregular shape, including but not limited to, dots, squares, circles, ellipses, continuous or discontinuous stripes, and so on.

The web according to the present invention comprises at least one region comprising particles of chitosan material on one or both of its surfaces (herein also called first and second surfaces) and/or between its surfaces. In the case where the region comprising particles of chitosan material is situated between the surfaces, said region can then be described as being spaced apart from the surfaces along the z-direction of said web.

Typically, at least one region of the web comprises particles of chitosan material at a level of from 0.1 g/m² to 200 g/m², preferably from 1 to 100 g/m², and more preferably from 2 to 50 g/m² of said web.

According to a preferred embodiment of the present invention at least one region comprising the particles of chitosan material extends across at least 40%, preferably 60%, more preferably 80% and most preferably 100% of the extension of said web in the plane formed between the x- and y-axis of the web.

As an essential feature the web according to the present invention comprises particles of chitosan material having a particle size distribution with a mean diameter D(v,0.9) of not more than 300 µm.

Preferably these particles have a particle size distribution with a mean diameter D(v,0.9) of not more than 100 µm and more preferably not more than 50 µm.

By 'mean diameter D(v,x) of less than y µm' for a particle size distribution it is meant that x*10% of the particles have a mean diameter of less than y µm. For instance, a D(v,0.9) of not more than 100 µm indicates that 90% of the particles of chitosan material have a mean diameter of not more than 100 µm.

The particle size of the chitosan material can be determined by ESEM analysis, using a Philips XL30 ESEM FEG electronic microscope for example. A sample of 1.5 cm x 1.5 cm was cut with a scissors from the central part of the web and mounted on a circular aluminium stub having a diameter of 1.2 cm, and then sputtered with a layer of gold having a thickness of 30 nm. The sample was observed in SEM mode *in vacuo* at an appropriate magnification to visually investigate the particle size of the chitosan material, taking six images in different zones of the sample.

The final coverage assessment is evaluated by visual grading (evaluation of number of covered fibres).

Importantly, the selection of these small particles of chitosan material (namely salts of chitosan material like chitosan amino salts) contributes to the outstanding leakage prevention towards fluids associated with the web of the present invention. Indeed these chitosan particles have been found to be more effective than bigger particles in various properties, namely in terms of their gelification properties and fluid retention properties.

It is to be understood herein that any method known to those skilled in the art to provide a web with the particles of chitosan material having the particles size distribution described herein is suitable to be used herein. This includes spraying processes, curtain coating, printing and slot coating processes. Highly preferred herein is to use a spraying process as described in more details herein after in the process for making a preferred web according to the present invention.

In a highly preferred embodiment herein, the particles of chitosan material are present onto said region of said web in a 'substantially homogeneous pattern'. This means that the particles of the chitosan material are in close physical proximity in said region of the web, which comprises the chitosan material. In other words, almost no space is present between adjacent particles of chitosan material, so that the chitosan material particles preferably form a substantially continuous film-like layer (see Figure 2). In a preferred embodiment herein wherein the applied latex underlies the pattern of chitosan material, the chitosan material covers not only the latex, which was applied onto the surface of the web prior to the application of the chitosan material, but also those parts of the web's surface, which do not comprise latex.

The web of the present invention can comprise further optional components. In a preferred embodiment, the web according to the present invention might comprise a latex binder.

In a particularly preferred embodiment, the web contains particulate superabsorbent polymeric material, such as anionic superabsorbent material like absorbent gelling material based on polyacrylates. The superabsorbent polymeric material suitable for use herein can be in the form of fibres or of powder.

Another class of optional compounds, which can be comprised by the web of the present invention, is odour control compounds. In particular, the web of the present invention can comprise silica, zeolites, pH-adjusting material, chelants like EDTA, metal ions, cyclodextrins, urease inhibitors, antimicrobial compounds, activated carbon and mixtures thereof.

### Process for producing a web according to the present invention

The process for making the chitosan-coated web of the present invention is characterized by the essential steps of forming a precursor web, and subsequently applying chitosan material onto at least one surface of the precursor web in the form of particular spray features.

Indeed in the process according to the present invention the chitosan material is applied onto the precursor web (so-called herein to distinguish it from the web resulting from the process described herein) as a solution or dispersion in the form of a spray of droplets having a particle size distribution with a mean diameter D(v,0.9) of less than 1500 µm, the amount of chitosan material solution or dispersion applied onto the web being preferably from 1 ml to 1000 ml per square meter of web.

It has now been found that by applying the solution or dispersion of chitosan material on a precursor web in the form of a spray of droplets having a particle size distribution with a mean diameter D(v,0.9) of less than 1500 µm, preferably less than 1000 µm, more preferably less than 750 µm, a substantially homogeneous pattern of the chitosan material is provided on the web which translates in excellent fluid handling and gelifying performance towards fluid, namely electrolyte-containing fluids, while requiring less amount of chitosan material. Indeed, by applying the chitosan material solution or dispersion onto the web in the form of a spray of small droplets as defined herein, a higher coverage of the surface sprayed is achieved, as compared to applying the same chitosan material solution or dispersion but in the form of a spray of droplets with larger droplets. Furthermore, applying the solution or dispersion of chitosan material on the web as mentioned herein, translates into limited wetting of the surface, and thus in faster drying of the chitosan material solution or dispersion. In other words, a substantially homogeneous pattern of chitosan material particles is generated in less time, resulting in improved process ability and hence reduced process costs.

By 'mean diameter D(v,x) of less than y µm' for a droplet size distribution it is meant that x*10% of the spray of droplets dispensed (expressed in volume unit) has a mean droplet diameter of less than y µm. For instance, a D(v,0.9) of less than 1500 µm indicates that 90% of the total sprayed volume is dispensed with droplets whose mean diameter is less than 1500 µm.

The particle size distribution of a spray of droplets can be determined by following the procedure detailed below:

Suitable test equipment is the Malvern Mastersizer S LongBed® with 1000 mm lens and a maximum particle size range of 3475 microns. The Malvern Mastersizer S LongBed® provides 21 cm opening (between lenses) to accommodate spray flow. In all readings at the Malvern® , the lens surface must remain free of spray contamination. In the present setup procedure, the distance from nozzle to laser was fixed at 8 cm, this to minimise lens contamination. At 8 cm distance, the spray was directed to the laser beam to place the laser centre to the spray cone. At least three readings have to be made for each sample of chitosan material solution/dispersion sprayed to determine the particle size distribution of the spray of droplets. The sprayer used in the test according to the present invention was an electrically operated sprayer.

Any apparatus adapted to deliver a spray of droplets as defined herein are suitable for use herein. Several modifications can be made to the conventional, single aperture, spray head to ensure that a spray of such droplets as required herein is formed. Suitable apparatuses to be used herein (also called spray dispensers) share the common feature of having at least one aperture or a plurality of apertures also called "dispensing openings" or "spray nozzles" through which the solution/dispersion of the chitosan material is dispensed already mixed with air, said apertures being configured so as to deliver the spray of droplets having the characteristics mentioned herein. Suitable apparatus for use herein are air atomizers or nebulizators being or not electrically operated.

According to the above-described spraying procedure a substantially homogeneous pattern of chitosan material particles is typically provided on the web, in which after drying the particles of chitosan material preferably have a particle size distribution with a mean diameter D(v,0.9) of not more than about 300 µm, preferably not more than about 100 µm and more preferably not more than 50 µm. This substantially homogeneous pattern of small particles of chitosan material is generated on the web with reduced amount of chitosan material.

In a preferred embodiment of the present invention, a substantially continuous, film-like layer of particles of chitosan material is formed, said layer having reduced thickness, preferably less than about 500 µm and more preferably less than about 200 µm.

In Figure 3, the small particle size (namely less than 300 µm) of the adjacent particles of chitosan material, when applied according to the process of the present invention, and the homogeneity of the pattern of the chitosan material particles on the web surfaces is illustrated.

The process for making an absorbent web according to the present invention comprises the steps of:
(a) forming a precursor web having a first and a second surface, said second surface being approximately aligned opposite to said first surface, and
(b) applying during process step (a) onto at least one surface of said precursor web a solution or dispersion comprising a chitosan material, or
(b') applying after process step (a) onto at least one surface of said precursor web a solution or dispersion comprising a chitosan material, said solution or dispersion is applied onto said precursor web in the form of a spray of droplets as described herein.

In a preferred embodiment herein, the process comprises subsequently to step (a) the additional steps of applying latex onto at least one surface of said precursor web and drying said web. After the drying, step (b') follows.

Several methods are known for applying latex binder to the precursor web, while spray bonding and print bonding are particularly preferred for webs, intended to be used in absorbent articles. The 'latex' is usually an aqueous dispersion and can be applied to a surface of the web. Preferably, the polymeric component of the aqueous latex for use in the present invention substantially consists of hydrophilic material.

The latex is applied as an aqueous emulsion or dispersion, which typically contains about 5 to 65% and preferably, 10% of solids and these materials are readily available from several manufacturers. Because the latex dispersions are water miscible, they may be further diluted, if desired, before being applied to the web. In addition, these latex compositions are thermosetting and in order to effect cross-linking, they can contain a small amount of suitable cross-linking agents which are well known chemical agents for this purpose, such as N-methylolacrylamide. Any type of latex known in the art can be used herein, if the polymeric component is substantially hydrophilic and the latex does not generate detectable odours, especially after curing, which would be unacceptable to the wearer. Latices available are classified by chemical family and those particularly useful herein include vinyl acetate and acrylic ester copolymers, ethylene vinyl acetate copolymers, styrene butadiene carboxylate copolymers and polyacrylonitriles and sold, for example, under the trade names of Airbond, Airflex and Vinac of Air Products, Inc., Hycar and Geon of Goodrich Chemical Co. and Fulatex of H. B. Fuller Company. A particularly preferred example of latex suitable for use in the present invention is Airflex 192, obtainable from Air Products and Chemicals Inc., Allentown, PA, USA. The amount of latex used in the web cannot be so high as to substantially impair or obscure the effective gelification capacity of the chitosan material and the absorbent properties of the hydrophilic fibres, or as to impart a degree of stiffness to the web as to render it impractical. The latex applied onto the web may range from about 1 to 30 g/m², preferably from about 1 to 20 g/m² and more preferably from 1 to 10 g/m² of the total basis weight of the web. Latex with substantially hydrophobic polymeric components can also be used in the present invention by rendering them hydrophilic after their application onto the web, e.g. by surfactants.

The presence of latex underlying the particles of chitosan material has the advantage of contributing to the control of the penetration of the chitosan particles into the web to which they have been applied to. Preferably, the chitosan particles do not penetrate more than 30%, preferably not more than 20% and more preferably not more than 10% of the calliper of the web. The presence of latex in the process of the present invention allows the production of highly preferred web according to the present invention that are even more effective in leakage prevention towards fluid while more efficiently using chitosan material on the surface of the web to which it is applied to.

In preferred embodiment, the web of the present invention is an air laid fibrous web.

The web of the present invention can be made using conventional equipment designed for dry laying processes and although the invention is described herein below with particular reference to air laid webs, it should be understood that other dry laying processes, e.g. carding, or other processes for creating fibrous substrates, such as the meltblown process or the spunbond process, or film forming or foaming are also applicable. Furthermore, it should be understood that the process as described hereinafter is not limiting the scope of the present invention. The following reference numerals refer to Figure 1.

In accordance with a preferred embodiment of the present invention, the precursor web is made by an air laid process. The air forming system includes a distributor unit (1) disposed transversely above a continuous forming screen (3) mounted on rollers and driven by a suitable motor (not shown) and a vacuum means or suction box (2) is positioned beneath the screen (3). In a conventional air forming system, upstream of the distributor unit is a defibrator or feeder (not shown), such as a hammer mill or Rando-Feeder, where bales, laps or the like are defiberized and further the fibres may be cleaned and/or blended if necessary or desired depending largely on the type of fibres used, the blend of fibres used and the end web sought. For example, wood pulp fibres can be blended with substantially hydrophilic synthetic fibres and applied as a blend by a single distributor, or different fibres can be each conveyed by a different distributor to the screen to form separate plies or layers.

The porous forming screen (3) is essentially coextensive with the distributors (1), and the suction box (2) beneath the screen (3) draws the air stream downwardly and conveys the fibres to the surface of the screen (3), thereby forming plies or a loose precursor web. At this stage in the process, the precursor web exhibits little integrity and the vacuum means (2) retains the loose, fibrous precursor web on the screen (3). The precursor web has a first surface that faces the distributor (1) and a second surface, opposite to said first surface, which faces the forming screen (3).

It should be understood that the system might be modified to control the composition and thickness of the end web. For example, the distributor unit (1) can comprise a plurality of individual distributors and this number of distributors and their particular arrangement can be altered or varied depending on such factors as machine speed, capacity, type of fibres and end web desired.

At this stage of the process, the precursor web on the screen (3) has very little integrity and requires stabilization. The precursor web is advanced by the continuous screen and if desired, the precursor web first may be passed between compression rollers (not shown), which may be heated, in order to increase the density of the precursor web, but this step is optional. This densification step enhances the penetration of the latex, which is applied subsequently onto the precursor web and the degree or percentage of densification can vary depending on such factors as the basis weight of the precursor web, the desired degree of penetration of the latex into the web of the present invention and the end web sought.

From there, the precursor web is transported to the first latex application section (4) having a suitable dispensing means, such as a spray nozzle, doctor blade, roller applicator, or the like, where a liquid dispersion of the latex binder is applied to the first surface of the loose precursor web. Optionally, a vacuum applied by a suction box (5) positioned beneath the dispensing means and the screen helps to draw the latex dispersion into the precursor web. The dispensing means or applicator is essentially coextensive with the width of the precursor web and preferably the latex is applied substantially homogeneously to the surface of the precursor web. However, the latex dispersion may be applied as a non-uniform or random application and because the latex dispersion is water-based, it will diffuse throughout the precursor web and function as a binder when cured.

The latex when cured imparts integrity to the precursor web and therefore some penetration of the latex is required. The extent or degree of penetration of the latex into the precursor web is controlled by the amount of latex applied and optionally by the vacuum applied to the precursor web in that the vacuum helps to draw the latex dispersion into the precursor web. The latex is a substantially hydrophilic thermosetting plastic and in order to activate the latex, the latex dispersion can contain a suitable curing agent or cross-linking agent and after the latex is applied onto the precursor web, the latex is cured to effect cross-linking. In a particularly preferred embodiment, curing of the latex is accomplished by passing the latex-treated precursor web after the first latex application section (4) through a first drying section (6), e.g. a hot air oven or an air drier. The temperature inside of the first drying section typically ranges from about 100 °C to 260°C, but this depends upon the specific type of latex resin used, upon the curing agent or cross-linking agent, upon the amount of latex, the thickness of the precursor web, the degree of vacuum and the machine speed. It is essential for the process according to the present invention that the first surface of the latex-treated precursor web is substantially dry after the first drying section (6).

After the first drying section (6), the precursor web is sprayed in the chitosan application section (7) with the solution or dispersion of a chitosan material onto the same surface, onto which latex was applied before. According to the present invention, the solution or dispersion of the chitosan material is applied on the precursor web in the form of a spray of droplets having a particle size distribution with a mean diameter D(v,0.9) of less than 1500 µm. The amount of chitosan material solution or dispersion applied onto the web is preferably from 1 ml to 1000 ml, more preferably from 1 ml to 400 ml and most preferably about 120 ml of solution or dispersion of chitosan material per square meter of web. For achieving the above-mentioned particle size distribution of said spray, it is particularly preferred to use so-called air atomizers or nebulizators for applying the solution of the chitosan material onto the surface of the precursor web. Examples therefore are the air atomising nozzles of the ¼ JAU series from Spraying Systems, Co., Wheaton, Illinois, USA. By such a spray process, after drying chitosan material particles having a particle size distribution with a mean diameter D(v,0.9) of not more than about 300 µm are generated on the surface of the precursor web, which was sprayed with the solution or dispersion of the chitosan material.

It is preferred to also apply latex to the second surface of the precursor web as well. Therefore, the precursor web is preferably reverted. After the reversion step, latex is applied onto the second surface of the precursor web by the second latex application section (8) in essentially the same way as the first surface in the first latex application section (4). In addition, the second latex application section (8) can include a suction box (9) for controlling the penetration of the latex into the precursor web. This second latex application is likewise cured by passing the precursor web through a second drying section (10) subsequent to the second latex application section (8) within about the same temperature range as indicated at the first drying section (6).

The resulting web of a preferred embodiment the present invention leaving the second drying (10) section subsequently passes a third drying (11) section for removing last traces of moisture and afterwards exhibits sufficient integrity and can be cut, rolled, packaged, etc.

In an alternative embodiment, the chitosan material can be sprayed onto the second surface of the precursor web after the second drying section (10), instead onto the first surface after the first drying section (6). For this purpose, the second surface of the precursor web has to be substantially dry after having passed the second drying section (10).

It is also within the scope of the present invention that the chitosan material is applied onto the first and/or second surface of the precursor web after the first (4) or the second (8) latex application sections, when the precursor web is still wet from the latex dispersion applied.

The concentration of the solution or dispersion of chitosan material to be applied onto the precursor web can vary from 0.1 to 40% by weight of chitosan material and is preferably from 1 to 10% by weight of chitosan material. The pH of the solution or dispersion of chitosan material to be applied onto the precursor web is between 3 and 7, preferably between 4 and 6 and more preferably 5 for an optimum match between skin-friendliness and water-solubility.

Notwithstanding the application of the latex, the web is soft yet strong and absorbent, exhibiting a relatively high tensile strength. It is desirable for preferred webs of this type to have relatively low bulk, because a more dense web, when compared to similar webs containing no latex and of about equal absorptive capacity but of higher bulk, can be thinner yet highly absorbent and consequently less bulky. A reduction in bulk, which means a reduction in volume the web is occupying, without sacrificing significantly other desired properties, is important from the standpoint of manufacturing, storage and packaging. Hence, for webs of this invention the basis weight ranges from about 50 g/m² to 400 g/m², preferably from about 75 g/m² to 350 g/m² and more preferably from 100 g/m² to 200 g/m². There can be manufacturing constraints in producing a web having a basis weight lower than about 50 g/m² in that such a web may lack desired strength. When the basis weight exceeds the upper limit, the web may be too stiff and therefore not useful for most applications.

Webs made according to the above-described embodiment of the present invention exhibit a good integrity due to the application of the latex, combined with good liquid barrier capabilities. The depth of penetration of the latex binder into the web is controlled by the choice of the polymer concentration and the amount of the latex to be applied onto the precursor web and optionally by the vacuum applied by means of the suction boxes positioned in correspondence with the dispensing means.

In an alternative embodiment of the web of the present invention, being further capable of absorbing aqueous fluids, a porous reinforcing sheet such as a creped paper, a tissue, or a nonwoven, can be incorporated into the precursor web either as a surface sheet or as an intermediate sheet disposed intermediate the first and second surfaces of the web. The sheet can be present on one surface of the web, while the opposite surface bears cured latex.

In an embodiment the precursor web can be a polyester or a polyolefin nonwoven onto which the air laid fibres can be layered by the fibre distributor, a bonding between the fibres and the sheet being achieved by means of mechanical entangling, while the latex binder is subsequently only applied to the surface of the precursor web which is opposite to the sheet.

As mentioned before, the use of a binder, such as latex, is optional in the process of the present invention. Therefore, the above-described embodiment of the process for making the web according to the present invention is in no way limiting the scope of the present invention.

In specific embodiments of the present invention, it is intended to make a web comprising particles of chitosan material between the two surfaces of the web. Such webs are preferably made by a process comprising the essential steps of forming a precursor web by e.g. air laying, applying chitosan material to said precursor web by a spray process as described herein above, and subsequently continue the web formation by e.g. further air laying onto the region of the precursor web, onto which the chitosan material has been sprayed.

The present invention is illustrated by the following example: An example of the preferred web is an airlaid substrate made of 68% of cellulose fibers mixed with 17% of superabsorbent powder comprising polyacrylic compounds, 11% of synthetic fibers, and 4% of latex (2% per surface) applied via spraying system, the particles of chitosan pyrrolidone carboxylate are sprayed as a solution of 4% by weight of chitosan pyrrolidone carboxylate in water onto one side of the airlaid web at a loading of 6 g of chitosan pyrrolidone carboxylate per square meter of the web after drying out the web. This web has been used for taking the pictures of figures 2 and 3.

## Claims

1. An absorbent web suitable for use in disposable absorbent articles, having a x-axis, a y-axis and a z-axis formed by an orthogonal Cartesian coordinate system, said web having a dimension extending along each of said x-, y-, and z-axis, said web further having a first surface extending in the plane formed between said x- and y-axis, and a second surface opposite to said first surface, said second surface being separated from said first surface by said z-dimension of said web, said web comprising at least one region between said first and second surfaces, said region comprising particles being particles of chitosan material, said web being **characterized in that** said particles have a particle size distribution with a mean diameter D(v,0.9) of not more than 300 µm.

2. A web according to claim 1, **characterized in that** said region comprises said particles of chitosan materials in a substantially homogeneous pattern.

3. Web according to any of the preceding claims, **characterized in that** said particles have a particle size distribution with a mean diameter D(v,0.9) of not more than 100 µm and preferably not more than 50 µm.

4. Web according to any of the preceding claims, **characterized in that** said web comprises a dry laid, preferably an air laid, fibrous web.

5. Web according to any of the preceding claims, **characterized in that** said region comprises particles of chitosan material in an amount of 0.1 to 200 g per square meter, preferably from 1 to 100 g per square meter and more preferably from 2 to 50 g per square meter of said web.

6. Web according to any of the preceding claims, **characterized in that** said chitosan material comprises at least one salt of chitosan, said salt preferably being chitosonium pyrrolidone carboxylate and/or chitosonium lactate.

7. Web according to any of the preceding claims, **characterized in that** said at least one region comprising particles of chitosan material extends across at least 40%, preferably 60%, more preferably 80% and most preferably 100% of the extension of said web in said plane formed between said x- and y-axis.

8. A disposable absorbent article, comprising a topsheet, a backsheet and an absorbent core positioned between said topsheet and said backsheet, **characterized in that** said core comprises a web according to any of the preceding claims.

9. Process for making an absorbent web, said process comprising the steps of:
(a) providing a precursor web having a first and a second surface, said second surface being approximately aligned opposite to said first surface, and
(b) applying during process step (a) onto at least one surface of said precursor web a solution or dispersion comprising a chitosan material, or
(b') applying after process step (a) onto at least one surface of said precursor web a solution or dispersion comprising a chitosan material, said process being **characterized in that** said solution or dispersion is applied onto said precursor web in the form of a spray of droplets, said droplets having a particle size distribution with a mean diameter D(v,0.9) of less than 1500 µm, preferably less than 1000 µm, more preferably less than 750 µm.

10. Process according to claim 9, said process comprising the additional steps of:
(a') applying latex onto at least one surface of said precursor web, and thereafter
(a") drying said precursor web, said process being **characterized in that** steps (a') and (a") are carried out after said step (a) and before said step (b').

11. Process according to claims 9 - 10, **characterized in that** said precursor web comprises a dry laid, preferably air laid, fibrous web.

12. Process according to claims 9 - 11, **characterized in that** said web comprises, after drying of said solution or dispersion of chitosan material, particles of chitosan material having a particle size distribution with a mean diameter D(v,0.9) of not more than 300 µm, preferably not more than 100 µm and more preferably not more than 50 µm, preferably in a substantially homogeneous pattern.

13. Process according to claims 9 - 12, **characterized in that** said chitosan material penetrates into said web to not more than 30%, preferably not more than 20% and more preferably not more than 10% by calliper of said web.

14. Process according to claims 10 - 13, **characterized in that** said latex is applied in an amount of from 1 to 30%, preferably from 1 to 20% and more preferably from 1 to 10% of the total basis weight of said web.

15. Process according to claims 9-14, **characterized in that** said chitosan material comprises at least one salt of chitosan, said salt preferably being chitosonium pyrrolidone carboxylate and/or chitosonium lactate.

16. Process according to claims 9 - 15, **characterized in that** said solution or dispersion of chitosan material is an aqueous solution or dispersion, comprising from 0.1 to 40% by weight of said chitosan material and preferably from 1 to 10% by weight of said chitosan material.

17. Process according to claims 9 - 16, **characterized in that** said solution or dispersion of chitosan material is applied in an amount of 1 ml to 1000 ml, preferably from 1 ml to 400 ml and more preferably from 1 to 150 ml of said solution or dispersion of chitosan material per square meter of said web.

18. Process according to claims 9 - 17, **characterized in that** said solution or dispersion of chitosan material is applied onto at least one surface of said precursor web across at least 40%, preferably 60%, more preferably 80% and most preferably 100% of the whole surface of said precursor web.

19. A chitosan-coated web, obtainable by a process according to any of claims 9 - 18.

20. Absorbent article comprising a web, obtainable by claim 19.
